# EUROPEAN PATENT APPLICATION

(11) **EP 1 016 717 A1**
(43) Date of publication of application: **05.07.2000**
(21) Application number: 97928479.1
(22) Date of filing: 26.06.1997
(51) Int. Cl.: C12N 15/12, C07K 14/82, C07K 16/18, C12N 1/21, C12N 5/16, C12N 15/11, C12P 21/02, C12Q 1/68, G01N 33/53, G01N 33/574

(54) **NOVEL PROTEIN REMARKABLY EXPRESSED IN HEPATIC CANCER, GENE ENCODING THE SAME, ANTIBODY THERETO, AND METHOD FOR DETECTING THE EXPRESSION THEREOF**

(71) Applicant: SUMITOMO ELECTRIC INDUSTRIES, LIMITED, Osaka 541 (JP)
(72) Inventor: KISHIMOTO, Toshihiko, Yokohama Works of Sumitomo, Yokohama-shi, Kanagawa 244 (JP); TAMURA, Taka-aki, Chiba Univer.,Faculty of Science, Chiba-shi Chiba 263 (JP); MAKINO, Yasutaka, Chiba Univer.,Faculty of Science, Chiba-shi Chiba 263 (JP)
(74) Representative: Baldock, Sharon Claire
(86) International application number: JP9702214
(87) International publication number: WO9900495

(57) **Abstract**

A novel protein (HRPI) showing increased expression in the course of carcinogenesis; a novel gene encoding this protein; the antisense gene of this gene; an antibody to the above-mentioned protein; and a method for monitoring hepatic cancer by using the above-mentioned protein, gene or antibody. Genes specifically expressed in rat hepatic cancer are extracted and one showing increased expression in hepatic cancer is isolated. After preparing its cDNA of the full length, the base sequence of this gene is determined to confirm that this gene is one specific to hepatic cancer. The amino acid sequence encoded by the above-mentioned gene is determined and a protein having this amino acid sequence is expressed in a recombinant *Escherichia coli* . Further, an antibody to the protein is prepared. By using this antibody, it has been clarified that the onset of hepatic cancer can be monitored by detecting the expression of the above-mentioned protein and detecting the above-mentioned gene.

## Description

### Technical Field

This invention relates to a protein expressed markedly in hepatic cancer, a gene encoding the protein, an antibody against the protein, and a method for detecting the expression of the protein or the gene.

### Background Art

Carcinoma (or cancer) is known to occur because of some abnormality in a gene. A change or abnormality in the gene at its transcription level, in particular, is regarded as a major cause of cancer ("Science" Vol. 222, 1983, pp. 765-771). To elucidate the mechanism of onset of carcinoma, the acquisition of a protein variedly expressed during carcinogenesis and a gene encoding the protein, or a protein differing in the state of expression among tissues and a gene encoding the protein has been performed eagerly since around 1980. For example, cancer-specific proteins, α-fetoprotein, and CEA are known which have been acquired by biochemically analyzing carcinomatous tissues, and searching for their differences from normal tissue.

Methods used to identify these substances comprised preparing monoclonal antibodies against a carcinomatous tissue, sorting antibodies reactive only to the cancer tissue from the resulting monoclonal antibodies, and then identifying substances which would serve as antigens reactive to the monoclonal antibodies.

Information obtained by these methods was mostly concerned with proteins, and no genes were obtained directly. Therefore, to acquire a gene from the protein obtained, the gene was selected from a population of genes, called a gene library, through a genetic engineering technique.

Concretely, this method of selection is described in "Molecular Cloning Second Edition" (Cold Spring Harbor Laboratory Press, 1989), chapters 8, 9 and 12. As shown there, the method prepares a so-called gene probe on the basis of information on the resulting protein, and acquires a gene corresponding to this gene probe from the gene library by a method called colony/plaque hybridization.

The same literature also shows in chapters 8, 9, 11 and 12 a method for screening a desired gene from the gene library by use of monoclonal antibodies.

With these methods, however, the desired protein should be large in amount, or the use of the monoclonal antibodies requires an antigen protein of the cell surface type with high antigenicity. A protein which obviates these requirements has been very difficult to obtain. Consequently, it has also been extremely difficult to acquire a gene encoding the protein.

The present invention uses methods entirely different from the foregoing some known methods, to isolate a gene whose expression in a hepatic cancer tissue is increased compared with a normal hepatic tissue.

Specifically, the present inventors disclosed a novel protein increasing in expression during carcigenosis and HRPI gene which was a novel gene encoding the protein in the Publication of International Application WO97/10333.

However, later on it was found out that the base sequence of the HRPI gene and the amino acid sequence of the HRPI protein had been in error. An object of the present invention is to provide a HRPI protein comprising the correct amino acid sequence, a gene encoding the protein, and an antibody against the protein.

### Disclosure of the Invention

To attain the above object, the present inventors conducted extensive studies. As a result, genes specifically expressed in rat hepatic cancer were extracted by the subtraction method, and a gene whose expression was increased in hepatic cancer was isolated by dot screening. From a hepatic cancer cDNA library, a full-length cDNA of the gene was obtained, and the base sequence of the gene was determined. Further, the amino acid sequence encoded by the gene was determined.

Moreover, Northern blot hybridization confirmed the cDNA to be a hepatic cancer specific gene. Also, a protein encoded by the cDNA was expressed in recombinant *Escherichia coli* (*E. coli*), and the protein was confirmed to have the same functions as those of its naturally occurring equivalent.

A novel rat protein HRPI specific to hepatic cancer and a gene encoding the protein were isolated by the above-described means.

Furthermore, the above protein was inoculated into a mammal other than a species from which the protein was derived (excluding human), to produce an antibody against the protein and confirm the antigenicity of the protein.

It was also found that the above gene was detected in respective tissues of the rat by Northern blot hybridization.

These findings showed that the onset and progress of cancer, especially hepatic cancer, could be monitored by detecting the expression of the protein by use of the above-mentioned antibody, and that the onset and progress of cancer, especially hepatic cancer, could be monitored by detecting the gene encoding the protein.

### Brief Description of the Drawings

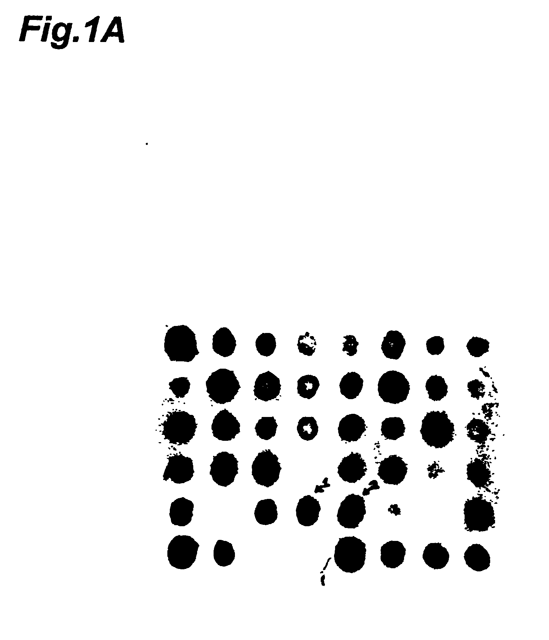

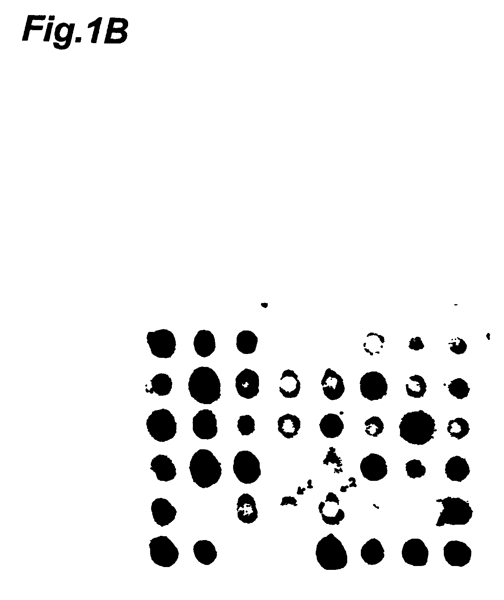

Figs. 1A and 1B are views showing the results of dot blot screening performed by fixing to a membrane, DNA that was extracted from a gene library obtained from hepatic cancer by the subtraction method in Example 1. Fig. 1A is a view showing the results of screening with a cDNA probe prepared from hepatic cancer polyA RNA, while Fig. 1B is a view showing the results of screening with a cDNA probe prepared from normal liver polyA RNA. Arrow 1 indicates a dot of a gene whose expression is increased in hepatic cancer, and arrow 2, a dot of a gene whose expression is increased in hepatic cancer.

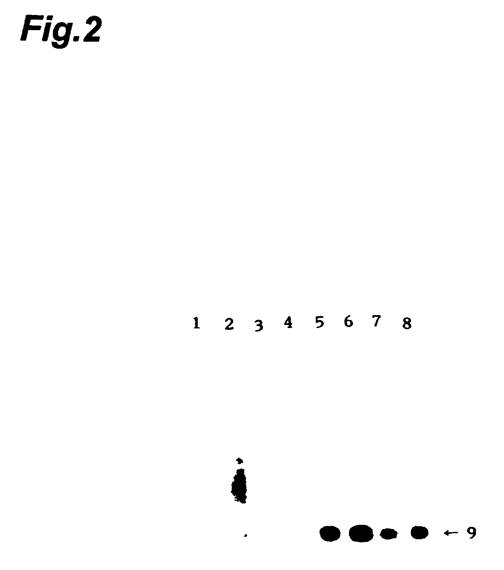

Fig. 2 is a view showing the results of analysis by Northern blot hybridization of mRNAs from normal liver and hepatic cancer with the use of HRPI gene as a probe. Lane 1 is a lane for normal liver mRNA; Lane 2 is a lane for mRNA of the liver 12 hours after administration of DEN; Lane 3 is a lane for mRNA of the liver 24 hours after DEN administration; Lane 4 is a lane for mRNA of the liver 48 hours after DEN administration; Lane 5 is a lane for mRNA of the hepatic cancer 1 month after DEN administration; Lane 6 is a lane for mRNA of the hepatic cancer 3 months after DEN administration; Lane 7 is a lane for mRNA of the hepatic cancer 5 months after DEN administration; Lane 8 is a lane for mRNA of the hepatic cancer 7 months after DEN administration; and arrow 9 indicates the position of the band of HRPI mRNA.

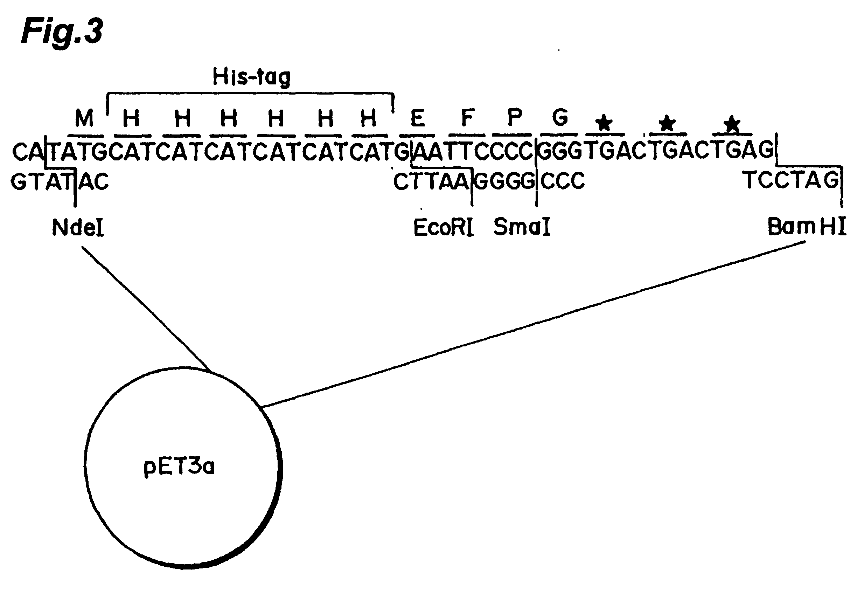

Fig. 3 is a view showing pET3a vector used in Example 4.

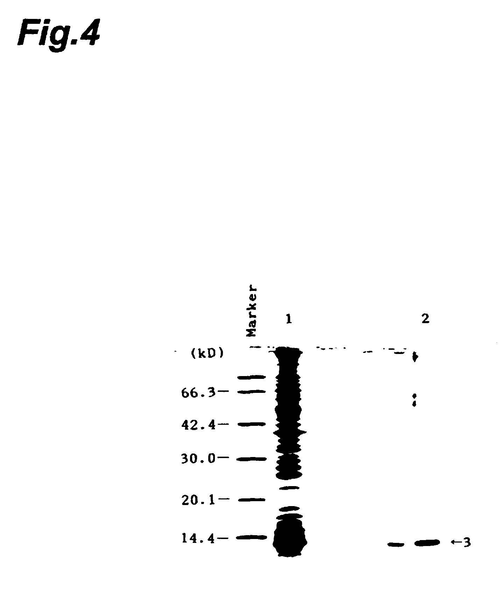

Fig. 4 is a view showing the results of SDS-PAGE electrophoresis of a recombinant HRPI partial protein. Lane 1 is a lane for a protein prepared by recombinant *E. coli*. Lane 2 is a lane for purified recombinant HRPI partial protein. Arrow 3 indicates the position of the band of the HRPI partial protein.

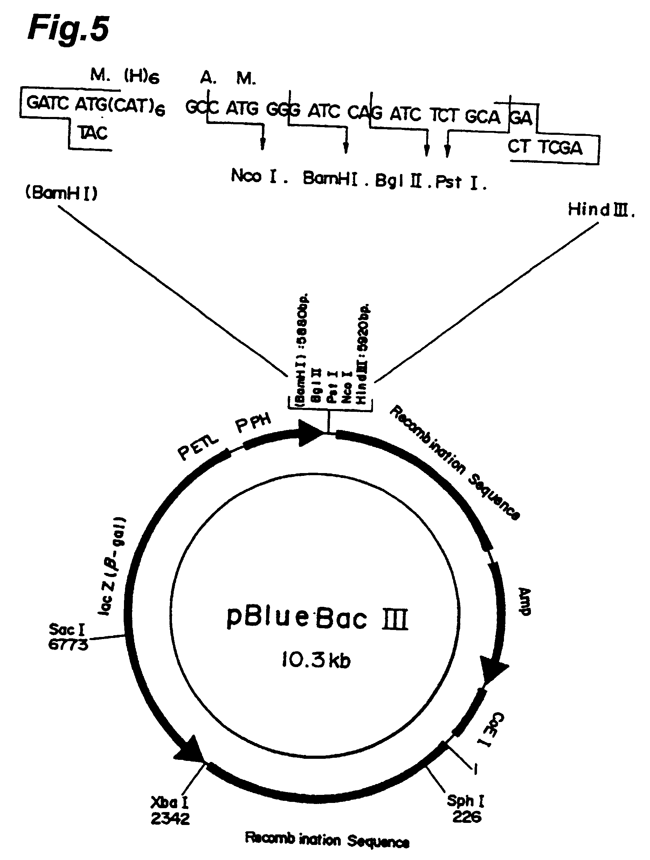

Fig. 5 is a view showing pBluebacIII vector used in Example 5.

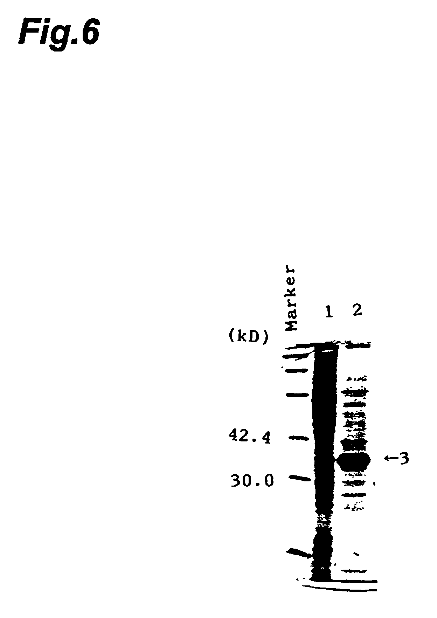

Fig. 6 is a view showing the results of SDS-PAGE electrophoresis of recombinant HRPI. Lane 1 is a lane for a protein expressed by wild type Sf9 cells. Lane 2 is a lane for a protein expressed by recombinant Sf9 cells. Arrow 3 indicates the position of the band of the recombinant HRPI.

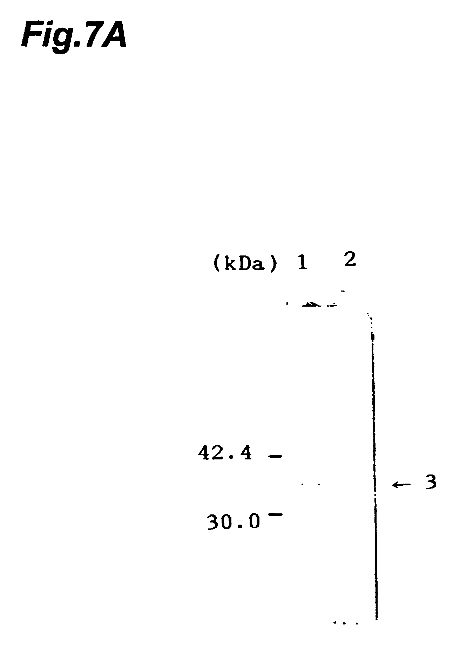

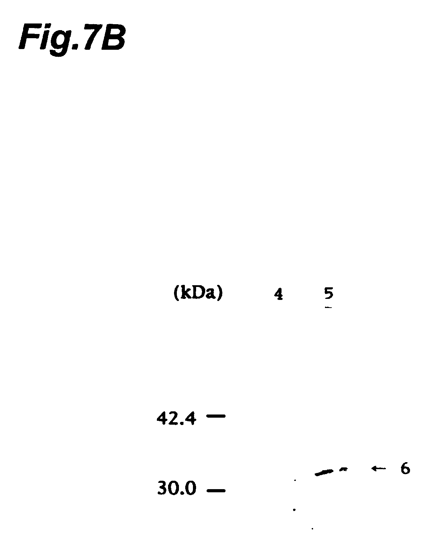

Figs. 7A and 7B are views showing the results of Western blot analysis of the reaction product between a sample containing HRPI full-length protein and anti-HRPI antibodies. Fig. 7A is a view showing the results of Western blot analysis of the reaction product between a sample containing recombinant HRPI full-length protein and anti-HRPI antibodies. Fig. 7B is a view showing the results of Western blot analysis of the reaction product between the extract of a rat liver and anti-HRPI antibodies. In Fig. 7A, Lane 1 is a lane for the electrophoresed recombinant HRPI, Lane 2 is a lane for the electrophoresed hepatic cancer tissue extract, and arrow 3 indicates the position of the band of the recombinant HRPI. In Fig. 7B, Lane 4 is a lane for the electrophoresed normal liver extract, Lane 5 is a lane for the electrophoresed extract of hepatic cancer 7 months after administration of DEN, and arrow 6 indicates the position of the band of HRPI.

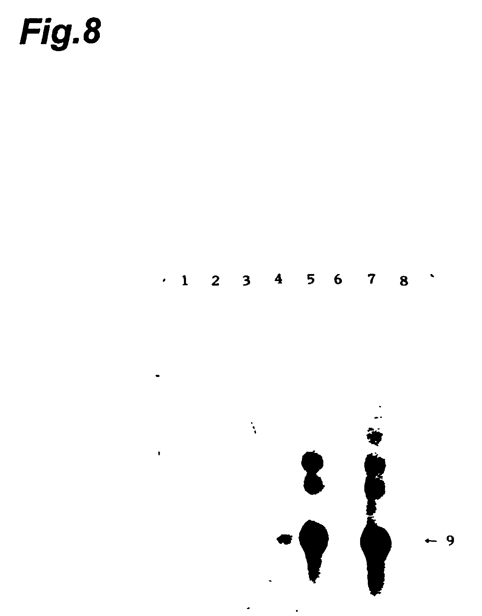

Fig. 8 is a view showing the expression of HRPI gene in various organs. Lane 1 is a lane for electrophoresed heart mRNA; Lane 2 is a lane for electrophoresed brain mRNA; Lane 3 is a lane for electrophoresed spleen mRNA; Lane 4 is a lane for electrophoresed lung mRNA; Lane 5 is a lane for electrophoresed liver mRNA; Lane 6 is a lane for electrophoresed skeletal muscle mRNA; Lane 7 is a lane for electrophoresed kidney mRNA; and Lane 8 is a lane for electrophoresed testicle mRNA. Arrow 9 indicates the position of the band of HRPI mRNA.

### Best Mode for Carrying out the Invention

The present invention concerns a protein whose expression is increased in a hepatic cancer tissue compared with a normal hepatic tissue, a gene encoding the protein, an antibody specific to the protein, and a method for monitoring cancer by detecting the protein or the gene.

The characteristics of the present invention are as follows:
1. A protein represented by an amino acid sequence described as SEQ ID NO: 1 in the Sequence Listing.
2. A protein comprising an amino acid sequence described as SEQ ID NO: 1 in the Sequence Listing wherein one or more amino acids have been added to, deleted from, or replaced for, and whose expression in a hepatic tissue is increased in cancer cells compared with normal cells.
3. The protein recited in Section 2, wherein mRNA encoding the protein is increased in cancer cells compared with normal cells at a level detectable by the subtraction method.
4. DNA comprising a base sequence encoding the protein recited in any one of Sections 1 to 3.
5. DNA comprising a base sequence encoding at least part of the protein recited in any one of Sections 1 to 3 and hybridizing to RNA encoding the whole protein.
6. DNA comprising a base sequence described as SEQ ID NO: 2 in the Sequence Listing.
7. DNA comprising a base sequence ranging from the 25th base A to the 912th base A in the base sequence described as SEQ ID NO: 2 in the Sequence Listing.
8. DNA comprising consecutive 12 bases or more in a base sequence encoding the protein recited in any one of Sections 1 to 3 and having a GC content of from 30 to 70%.
9. DNA comprising consecutive 16 bases or more in a base sequence encoding the protein recited in any one of Sections 1 to 3 and having a GC content of from 30 to 70%.
10. DNA comprising consecutive 12 bases or more in the -base sequence described as SEQ ID NO: 2 in the Sequence Listing and having a GC content of from 30 to 70%.
11. DNA comprising consecutive 16 bases or more in the base sequence described as SEQ ID NO: 4 in the Sequence Listing and having a GC content of from 30 to 70%.
12. The DNA recited in any one of Sections 4 to 11, which has been chemically modified.
13. Antisense DNA to the DNA recited in any one of Sections 4 to 11.
14. RNA comprising a base sequence encoding the protein recited in any one of Sections 1 to 3.
15. RNA comprising consecutive 12 bases or more in a base sequence encoding the protein recited in any one of Sections 1 to 3 and having a GC content of from 30 to 70%.
16. RNA comprising consecutive 16 bases or more in a base sequence encoding the protein recited in any one of Sections 1 to 3 and having a GC content of from 30 to 70%.
17. The RNA recited in any one of Sections 14 to 16, which has been chemically modified.
18. Antisense RNA to the RNA recited in any one of Sections 14 to 16.
19. An antibody specifically reactive to the protein recited in any one of Sections 1 to 3.
20. A method for detecting the protein recited in any one of Sections 1 to 3, the method using the antibody recited in Section 19.
21. A method for detecting cancer, comprising detecting the protein recited in any one of Sections 1 to 3, which is present in a tissue of a mammal, by use of the antibody recited in Section 19.
22. The method for detecting cancer recited in Section 21, wherein the tissue of a mammal is a hepatic tissue.
23. A method for detecting RNA encoding the protein recited in any one of Sections 1 to 3, the method using the DNA recited in any one of Sections 4 to 12 as a probe.
24. A method for detecting cancer, comprising detecting RNA encoding the protein recited in any one of Sections 1 to 3, which is present in a tissue of a mammal, by use of the DNA recited in any one of Sections 4 to 12 as a probe.
25. The method for detecting cancer recited in Section 24, wherein the tissue of a mammal is a hepatic tissue.

The present invention concerns a protein specifically expressed in hepatic cancer and a gene encoding the protein. As disclosed in the Examples to be described later on, they were obtained by isolating a gene, which is present specifically in hepatic cancer, from the liver of a rat by the subtraction method, and determining its base sequence. From the resulting gene, a protein expressed specifically in hepatic cancer was confirmed and isolated.

The present inventors have designated the protein specific to hepatic cancer as rat HRPI. The amino acid sequence of the protein and the gene sequence encoding it are shown in SEQ ID NO: 1 and NO: 2 in the Sequence Listing.

Heterologous proteins with the same function are generally known to have homology of 30-40% or more, although they are somewhat different in amino acid sequence depending on species. It can be fully presumed, therefore, that mammals other than rat also have a protein having the same function as the protein isolated in the present invention (HRPI), and having homology of 30% or more (preferably 40% or more) to said protein. As used herein, the presence of homology is generally determined by the calculation method which counts homologous amino acids as positive. When two proteins are different in the length of the amino acid chain, the homology rate is expressed as the proportion of the homologous portion to the length of the protein with a shorter amino acid chain.

Thus, the proteins according the present invention are intended to mean those having the following two features:
(1) In a hepatic tissue, expression in hepatic cancer is markedly increased compared with expression in normal cells.
(2) There is homology of 30% or more, preferably 40% or more, to the base sequence described in the Sequence Listing. Furthermore, this feature does not depend on the animal species of origin.

To know whether the protein is specifically observed (expressed) in hepatic cancer, various known methods are usable. For example, those in the Examples to be described later on are preferred methods for use in the confirmation.

Spontaneous or artificial mutation (see, e.g., Molecular Cloning 2nd Edition, Cold Spring Harbor Laboratory Press, 1989, pp. 15.1-15.113) can alter part of the structure of a polynucleotide, and accordingly, can mutate a protein encoded by the polynucleotide. In regard to the protein of the present invention, too, it is possible to produce a mutant protein in which one or more amino acids of the amino acid sequence described as SEQ ID NO: 1 in the Sequence Listing have been replaced for or deleted from, or in which one or more amino acids have been added to the amino acid sequence described as SEQ ID NO: 1 in the Sequence Listing. The proteins of the present invention may be specifically recognized by antibodies to the proteins, so that mutant proteins of the present invention may be characterized by being recognized by the antibodies (i.e., being mutated to the degree recognizable by the antibodies).

The genes encoding HRPI according to the present invention are genes coding for the HRPI defined above.

The present invention reveals the antigenicity of the above-mentioned protein, i.e. HRPI, such that the protein easily yields antibodies when used to immunize mammals other than the species of origin and other than human, as will be exemplified in the Examples. Hence, the antibodies of the present invention against HRPI include, within their scope, antisera and polyclonal antibodies which are obtained by a similar method, namely, immunization of an animal other than the species from which the protein was derived. The use, as an immunogen, of part of the protein or part of the protein bound to a carrier protein such as bovine serum albumin is a method in common use by those skilled in the art. The part of the protein may be synthesized by a peptide synthesizer, for example. Preferably, the part of the protein is 8 amino acid residues or more so that it may be allowed to function satisfactorily as an immunogen.

If polyclonal antibodies against the substance whose antigenicity has been revealed are obtained by immunization, monoclonal antibodies can be produced by hybridomas using lymphocytes of the immunized animal (e.g., see Antibodies, A Laboratory Manual, Cold Spring Harbor Laboratory Press, 1988, Chapter 6). Thus, the antibodies of the present invention against HRPI include polyclonal antibodies and monoclonal antibodies within their scope.

Methods for detecting HRPI include, for example, the use of antibodies, the use of antibodies relying on an enzyme reaction, and the detection of respective genes.

Methods using antibodies are concretely (1) a method for detecting each of the aforementioned proteins by use of antibodies to the protein, and (2) a method for detecting each protein by using antibodies against the protein and labeled secondary antibodies against the antibodies. The labels are, for instance, radioisotopes (RI), enzymes, avidin or biotin, and fluorescent materials (e.g., FITC and Rhodamine).

Methods using antibodies relying on an enzyme reaction include, for example, ELISA.

Methods for detecting genes are, for example, Northern blot hybridization, RT-PCR (Current Protocols in Molecular Biology, Greene Publishing Associates and Wiley-Interscience, Chapter 15), and in situ hybridization (*ibid*. Chapter 14).

The optimum conditions for hybridization are known to be different according to the length of the probe or the membrane used. What conditions to be set can be easily chosen by those skilled in the art.

The Examples of the present invention disclose optimum conditions worked out in view of the nature of the membrane used and the length of the probe aimed at. If the membrane and the probe length differ, hybridization, needless to say, can be achieved under different hybridization conditions. For example, hybridization may be effected in the absence of sodium pyrophosphate. A preferred embodiment is as follows:
(1) Prehybridization conditions
   5-10 x SSC (the concentration of each component of SSC solution is 5 to 10 times)
   5-10 x Denhardt's solution
   Not more than 1 M sodium pyrophosphate (pH 6.8)
   30-50% formamide
   0.1-1% SDS
   About 100 µg/ml yeast tRNA
   About 100 µg/ml thermally denatured DNA
   Reaction temperature: 35 to 42 °C
   Reaction time: from 50 minutes to 1 hour and 10 minutes
(2) Hybridization conditions
   5-10 x SSC
   5-10 x Denhardt's solution
   Not more than 1 M sodium pyrophosphate (pH 6.8)
   30-50% formamide
   Not more than 1% SDS
   About 100 µg/ml yeast tRNA
   About 100 µg/ml thermally denatured DNA
   1x10⁵ to 2x10⁶ cpm/ml cDNA probe
   Reaction temperature: 35 to 42 °C
   Reaction time: 12 to 20 hours

Of the above conditions, sodium pyrophosphate (pH 6.8) or SDS is mainly used for improvement based on the background. The probe for use in detecting these genes may be DNA or RNA.

The number of bases of the human genome is said to be 3x10⁹. Since DNA of 16 bases comes in 4¹⁶ types, this length of DNA enables human proteins to be all recognized.

That is, the length required of a probe is, theoretically, 16 bases. For practical purposes, a length longer than this is desirable, needless to say. Practically, a length of 12 bases or more is often used because of synthesis efficiency, operability and so on. The site used as the probe may be a noncoding region or a coding region.

If the site used as the probe has a GC content of 30 to 70%, it may be a noncoding region or a coding region.

The antisense DNA or antisense RNA of the present invention (which may be collectively called the antisense polynucleotide) includes all of nucleotides comprising bases, phosphates and sugars combined in a plurality of numbers, including those which are not present in nature.

The antisense polynucleotide derivative of the present invention includes all of those which are similar to polynucleotide in conformation and function. Examples are polynucleotides linked at the 3'-terminal or 5'-terminal to other substances, polynucleotides having undergone modification, such as replacement, deletion, or addition, in at least part of the bases, sugars or phosphates, polynucleotides having bases, sugars or phosphates that do not exist in nature, and polynucleotides having a skeleton other than sugar-phosphate skeleton.

The antisense polynucleotide or its derivative may be the one which hybridizes to any portion of a polynucleotide encoding the protein of the present invention whose expression is increased in hepatic cancer. The antisense polynucleotide or its derivative is preferably the one which has a base sequence complementary for part of mRNA encoding all or part of the protein, and which hybridizes to the mRNA. The particularly preferred one is that which hybridizes to mRNA encoding at least HRPI.

The antisense polynucleotide or its derivative is immediately usable as a polynucleotide probe for research use in investigating the presence and the status of expression, in a tissue or cells, of a polynucleotide encoding the protein of the present invention whose expression is increased in hepatic cancer. The antisense polynucleotide or its derivative is also usable as a diagnostic polynucleotide probe. The probe preferably has 12 bases or more and a GC content of 30 to 70%, more preferably, 16 bases or more and a GC content of 30 to 70%.

It is also possible to adjust the expression of the protein of the present invention whose expression is increased in hepatic cancer, by using the antisense polynucleotide or its derivative. This antisense polynucleotide or derivative is expected to prevent the expression of the protein by hybridizing to a gene or mRNA encoding the protein. Thus, it can be used as a therapeutic agent for a disease resulting from the disorder of function which the protein takes part in. That is, an antisense pharmaceutical can be developed from the antisense polynucleotide or its derivative.

Generally, preferred examples of an antisense polynucleotide derivative are known to be a derivative with enhancement in at least one of nuclease resistance, tissue selectivity, cell permeation, and binding capacity. Most preferably, the polynucleotide derivative is known to be the derivative having a phosphorothioate bond as a skeletal structure. The polynucleotide and its derivative of the present invention also include derivatives having such function or structure.

The antisense polynucleotide derivative of the present invention can be prepared, for example, by the method described in Antisense Research and Applications (CRC Publishing, Florida, 1993).

Wild type DNA or RNA, for example, can be synthesized by a chemical synthesizer. Alternatively, the antisense polynucleotide derivative of the present invention can be obtained by PCR using as a template a gene encoding the protein of the invention whose expression is increased in hepatic cancer. Some of the derivatives of the methyl phosphonate type or the phosphorothioate type can be synthesized by a chemical synthesizer (e.g., Model 394 available from Perkin-Elmer Japan). In this case, manipulations are performed in accordance with an instruction manual attached to the chemical synthesizer, and the resulting synthetic product is purified by HPLC using a reversed phase chromatograph, whereby the desired polynucleotide or polynucleotide derivative can be obtained.

When DNA or RNA is chemically or enzymatically synthesized, it is well known to subject it to a chemical modification, such as methylation or biotinylation of the side chain, or substitution of oxygen of the phosphate portion by sulfur.

Main examples of the modification to be introducible during chemical synthesis are DNA and RNA syntheses characterized by: 1) biotinylation, 2) methylation, 3) conversion to digoxigenin derivative, 4) dephosphorylation, 5)fluorescent labeling (fluorescein, Rhodamine, Texas Red and derivatives thereof), 6) amination, and 7) substitution of O of the phosphate group by S.

Main examples of chemical modification to be introducible enzymatically are 1) biotinylation, 2) methylation, 3) conversion to a digoxigenin derivative, 4) dephosphorylation, 5)fluorescent labeling (fluorescein, Rhodamine, Texas Red and derivatives thereof), and 6) enzyme labeling (alkaline phosphatase).

When DNA described as SEQ ID NO: 2 in the Sequence Listing is to be chemically synthesized, for example, it is possible to synthesize DNA different from the DNA of the Sequence Listing itself by performing the chemical modification described above. Thus, the DNAs and RNAs of the present invention include within their scopes the chemically modified DNAs and RNAs.

Monitoring of the progress of cancer by detection of HRPI can be performed by examining whether the protein is present or not in a tissue or cells sampled from a subject. If HRPI is secreted or liberated outside cells, the progress of cancer can be monitored by examining whether HRPI is present or absent in the blood of the subject. Concretely, the monitoring may be done in the same manner as the aforementioned method for detecting HRPI (the method using antibodies, the method using antibodies relying on enzyme reaction, or the method detecting the relevant genes).

Monitoring of the progress of cancer by the use of gene can be done by checking for the gene in a tissue or cells sampled from the subject. The methods for detecting the gene include, for example, Northern blot hybridization, RT-PCR, and in situ hybridization, as stated previously.

The present invention will now be described in detail by reference to Examples, which do not limit the present invention.

### [Example 1] Protein Increasing in Expression in Hepatic Cancer and Its Gene

### I. Isolation of gene encoding a protein whose expression is increased in hepatic cancer

### 1. Preparation of hepatic cancer rats

Hepatic cancer-bearing rats were prepared based on the Salt-Farber method (Nature, Vol. 263, 1976, pp. 701-703). Actually, 5-week Wistar rats (Funabashi Farm) were intraperitoneally administered diethylnitrosamine (DEN). Two weeks later, oral administration of M Feed (Oriental Yeast) containing 0.02% of 2-aminoacetylfluorene (AAF) was started. One week later, a regenerative hepatic operation was performed. The livers were removed 12, 24, 48 hours and 1, 3, 5 and 7 months after DEN administration, and used for subsequent RNA preparation.

As controls, regenerated livers showing normal growth were acquired by removing the livers 12, 24 and 48 hours after regenerative hepatic operation. These livers were used later for analysis.

### 2. Preparation of RNA

Total RNA was prepared based on the method described in Methods in Enzymology, Vol. 154 (Academic Press Inc., 1987), pp. 3-28. The actual procedure was as follows:
(1) Three grams of each liver was ground in liquid nitrogen, and added to 100 ml of a 5.5 M GTC solution (5.5 mM guanidine thiocyanate, 0.5% N-lauronylsarcosine, 25 mM sodium citrate, pH 7.0). The mixture was homogenized with a Potter type homogenizer.
(2) The homogenate was centrifuged for 10 min at 3,000 rpm, and then the supernatant was overlaid on 12 ml of a cesiumtrifluoroacetic acid solution (50% cesiumtrifluoroacetic acid (Pharmacia), 100 mM disodium ethylenediaminetetraacetate (EDTA) (pH 7.0) with a specific gravity of 1.6 g/ml that had been added in an SW28 swing rotor centrifuge tube (Beckman). The system was separated by the SW28 swing rotor for 24 hours at 25,000 rpm at 15 °C.
(3) The precipitate was dissolved in 600 µl of 4M GTC solution, and centrifuged at 15,000 rpm, whereafter the solution portion was recovered. Then, 15 µl of 1M acetic acid and 450 µl of ethanol were added, and the mixture was centrifuged for 10 minutes at 15,000 rpm, followed by recovering the precipitate.
(4) The precipitate was dissolved in a suitable amount (about 3 ml) of a TE solution (1 mM Tris-Cl (pH 7.5), 1 mM EDTA) (TE solution was added until the precipitate was dissolved). The solution was centrifuged at 15,000 rpm, and the solution portion was recovered.
(5) Phenol/chloroform in the same amount as the amount of the solution was mixed with the solution. The mixture was centrifuged for 10 minutes at 15,000 rpm, and the solution portion was recovered.
(6) To the solution, a 1/10 volume of 3 M sodium acetate (pH 5.2) was added, and ethanol in a 2.5-fold amount was added. The mixture was allowed to stand for 20 minutes at -20 °C, and then centrifuged at 15,000 rpm. The precipitate was washed with 70% ethanol, and then dried, dissolved in a suitable amount of TE solution, and stored at -80 °C. From each liver, 5-7 mg of total RNA was obtained.
(7) PolyA RNA was prepared by the use of oligotex dT30 super™ (Nippon Roche) in accordance with its instruction manual. The amount of the total RNA used was 1 mg per cycle, and polyA RNA was purified with 750 µl of oligotex dT30 super™. In each liver, about 20 µg of polyA RNA was obtained from 1 mg of the total RNA.

### 3. cDNA subtraction

This method was performed in accordance with the method of Hara et al. (Analytical Biochemistry, Vol. 214, 1993, pp. 58-64 (this reference is incorporated herein by reference).
(1) The polyA RNA to be used originated from each of the 7-month hepatic cancer and the normal liver, and 15 µg each was used.
(i) The polyA RNAs were each adsorbed by oligotex dT30 super™, and then the synthesis of cDNA was performed in this state. The conditions for the synthetic reaction followed the literature.
(ii) To the cDNA-oligotex dT30 super™ from hepatic cancer, poly dC tail was added using terminal deoxytransferase (Takara Shuzo), and cDNA for a sense strand was synthesized by means of EcoRI-(dG)₁₅ primer and Taq polymerase (Perkin-Elmer).
Between the sense strand cDNA and the cDNA-oligotex dT30 super™ from the normal liver, the cDNA subtraction reaction was carried out.
(iii) After the reaction, the resulting cDNA solution was amplified by a PCR reaction using both of primers, EcoRI-(dG)₁₅ and XhoI-(dT)₃₀, in accordance with the method described in Current Protocols in Molecular Biology (1987, Greene Publishing Associates and Wiley-Interscience), Chapter 15. The conditions for the PCR were 25 cycles, each cycle comprising three stages at different temperatures, 94 °C for 90 seconds, then 55 °C for 2 minutes, and then 72 °C for 3 minutes, using the solution of the following composition:

| | |
|---|---|
| cDNA solution | 69 µl |
| 10xTaq buffer (Perkin-Elmer) | 10 µl |
| 1.25 mM dNTP | 16 µl |
| EcoRI-(dG)₁₅ primer (2 µg/ l) | 2 µl |
| XhoI-(dT)₃₀ primer (2 µg/ l) | 2 µl |
| Taq polymerase (Perkin-Elmer)(5 U/µl) | 1 µl |
| | Total 100 µl |

(2)
(i) After subtraction treatment, the resulting gene library was digested with EcoRI (Takara Shuzo). The reaction system employed the following conditions:

| | |
|---|---|
| Gene solution | 10 µl |
| 10xH buffer (Takara Shuzo) | 10 µl |
| EcoRI (Takara Shuzo) | 5 µl |
| Sterilized water | 75 µl |
| | Total 100 µl |

Reaction temperature: 37 °C
Reaction time: overnight

(ii) After cleavage with EcoRI, 100 µl phenol/chloroform was mixed with EcoRI-digested genes, and the mixture was centrifuged at 15,000 rpm, followed by recovering of the aqueous solution portion. To the solution, 10 µl of 3 M sodium acetate (pH 5.2) was added, and 250 µl of ethanol was further added. Then, the mixture was centrifuged at 15,000 rpm, and the precipitate was recovered.
(iii) The recovered precipitate was washed with 1 ml of 70% ethanol, dried, and then dissolved in 75 µl of sterilized water. The solution was formed into the composition described below, which was reacted overnight at 37 °C for cleavage with XhoI.

| | |
|---|---|
| Gene solution | 75 µl |
| 1% BSA (Takara Shuzo) | 10 µl |
| 10xH buffer (Takara Shuzo) | 10 µl |
| XhoI (Takara Shuzo) | 5 µl |
| | Total 100 µl |

Reaction temperature: 37 °C
Reaction time: overnight

(3) Then, 100 µl phenol/chloroform was mixed with the system, and the mixture was centrifuged at 15,000 rpm, followed by recovering of the aqueous solution portion. To the solution, 10 µl 3 M sodium acetate (pH 5.2) was added, and 250 µl ethanol was further added. Then, the mixture was centrifuged at 15,000 rpm, and the precipitate was recovered. The precipitate was washed with 1 ml of 70% ethanol, dried, and then dissolved in 100 µl of sterilized water to prepare a gene library solution.

### 4. Incorporation of gene after subtraction into vector

(1) pBluescriptII vector (Stratagene) was digested with EcoRI and XhoI (Takara Shuzo). The digesting conditions were the same as those described in 3.(2).
(2) The digested ends of the digested pBluescriptII vector were dephosphorylated by bacterial alkaline phosphatase (Takara Shuzo) for 1 hour at 65 °C and then 100 µl of phenol/chloroform was mixed with the system, and the mixture was centrifuged at 15,000 rpm, followed by recovering the aqueous solution portion.
(3) To the solution, 10 µl of 3 M sodium acetate (pH 5.2) was added, and 250 µl of ethanol was further added. Then, the mixture was centrifuged at 15,000 rpm, and the precipitate was recovered. The precipitate was washed with 1 ml of 70% ethanol, dried, and then dissolved in sterilized water to a concentration of 100 ng/µl.
(4) The gene library solution obtained in 3. and the digested, dephosphorylated pBluescriptII vector were mixed and reacted in accordance with an instruction manual attached to ligation pack™ (Nippon Gene) to insert each gene of the library into the vector.

### 5. Incorporation of gene after subtraction into E. coli

In accordance with a standard method, the reaction mixture in 4.(4) was completely mixed with competent cells of *E. coli* JM109 (Takara Shuzo), and reacted for 30 minutes on ice, for 45 seconds at 42 °C, and for 3 minutes on ice. Then, 900 l of SOC culture medium was added, and the mixture was allowed to stand for 1 hour at 37 °C to incorporate the vector into the *E. coli* JH109. Then, the *E. coli* JM109 was recovered.

### 6. Extraction of gene

(1) This *E. coli* was sprinkled over a LB agar medium with the following composition, and cultured overnight to form colonies.
Composition of LB agar culture medium:

| | |
|---|---|
| Ampicillin (Wako Pure Chemical) | 100 µg/ml |
| IPTG (Takara Shuzo) | 0.1 mM |
| X-gal (Takara Shuzo) | 0.004% |

Of the colonies formed, white colonies were selected as inoculum for use in gene screening to be performed later on.
(2) Two thousand kinds of inocula were selected, and were each cultured in a 2 ml LB liquid medium containing 100 µg/ml ampicillin. Then, genes were extracted by the alkali method described in Molecular Cloning Second Edition (Cold Spring Harbor Laboratory Press, 1989), Chapter 1.

### 7. Dot blot screening

(1) The extracted genes were each blotted to two nylon membranes (Millipore) using Bio Dot (Bio-Rad). After denaturation with sodium hydroxide aqueous solution, the genes were fixed with a UV Cross-Linker (Stratagene). Denaturation of the genes was carried out under the following conditions.
   (i) The genes were each allowed to react with a solution of 0.1 M sodium hydroxide and 0.15 M sodium chloride for 20 seconds.
   (ii) Then, the system was allowed to react with a solution of 0.2 M Tris-Cl (pH 7.5) and 0.15 M sodium hydroxide for 2 minutes.
   (iii) Then, the system was reacted in 2xSSC for 2 minutes.
(2) The hepatic cancer polyA RNA and normal liver polyA RNA prepared in 2. were subjected to a reverse transcription reaction with AMV reverse transcriptase (Seikagaku Corporation) using radioactive CTP (α-³²P-dCTP) (Amersham) to prepare cDNA probes from the respective polyA RNAs.
(3) The genes fixed to the nylon membranes in (1) were each hybridized to each of the cDNAs prepared in (2) under the following conditions:
   (i) Prehybridization conditions
      5xSSC
      5xDenhardt's solution
      0.1 M sodium pyrophosphate (pH 6.8)
      50% formamide
      0.5% SDS
      100 µg/ml yeast tRNA
      100 µg/ml denatured salmon sperm DNA
      Reaction temperature: 42 °C
      Reaction time: 1 hour
   (ii) Hybridization conditions
      5xSSC
      5xDenhardt's solution
      0.1 M sodium pyrophosphate (pH 6.8)
      50% formamide
      0.5% SDS
      100 µg/ml yeast tRNA
      100 µg/ml denatured salmon sperm DNA
      cDNA probe prepared in (2) (5x10⁵ cpm/ml)
      Reaction temperature: 42 °C
      Reaction time: 16 hours
(2) Then, the nylon membranes were each washed with 500 ml of 2xSSC (containing 0.1% SDS), 0.2xSSC and 0.1xSSC in this order, each for 30 minutes at 60 °C, and then subjected to autoradiography. The results are shown in Fig. 1. Based on the resulting autoradiograms, selection was made of the genes bound in larger amounts to the hepatic cancer-derived cDNA probe than to the normal liver-derived cDNA probe. At least 31 were confirmed in total. One out of them was selected, and used for the experiments described below.

### 8. Base sequencing

Determination of the base sequences was performed in accordance with the method described in Molecular Cloning Second Edition, Chapter 13. Actually, the base sequencing of the gene bound in a large amount to the hepatic cancer cDNA probe was carried out by reading the sequence of the gene portion inserted onto the pBluescriptII by the dideoxyterminator method using T7sequence kit™(Pharmacia).

### 9. Analysis of homology

The determined base sequence of the gene was analyzed for homology by referring to the data bank of DDBJ (DNA Database of Japan). As a result, the gene was found to be a novel gene without any homology. The gene was then named "HRPI gene." The gene was analyzed in the analysis described below to confirm whether its length was full-length.

### 10. Construction of cDNA library

From 4 µg of the polyA RNA extracted from the hepatic cancer tissue 7 months after DEN administration, cDNA was synthesized by means of Pharmacia's Time Saver cDNA Synthesis Kit™ in accordance with its instruction manual. The outline of this synthesis is provided below.
(1) Reverse transcription reaction was performed using a random primer, and a DNA synthesis reaction was carried out with DNA polymerase to synthesize double-stranded cDNA. To add NotI/EcoRI adapters to both ends of the cDNA, the cDNA was treated with T4 DNA ligase and polynucleotide kinase. As a result, cDNA having EcoRI restriction enzyme cleavage sites at both ends was obtained.
(2) This cDNA was inserted into a λgt11 cloning vector (Pharmacia) by use of T4DNA ligase, and packaged with GIGAPACK Gold™(Stratagene). Thus, the cDNA was incorporated into the λphage for isolation of the gene.

### 11. Isolation of gene

Isolation of the gene was performed in accordance with the method described in Molecular Cloning Second Edition, Chapter 2. The outline is provided below.
(1) The cDNA-containing library constructed in 10. was contacted with Y1090r-*E. coli*, then mixed with a NZY medium containing 0.7% agar, and sprinkled on a NZY medium plate containing 1.5% agar. Incubation was performed for 6 hours at 42 °C to form plaques containing a large amount of cDNA. Then, a nitrocellulose filter (Immobilon, Millipore's registered trademark, Millipore) was placed on the plate to transfer the plaques onto the filter.
(2) The filter was treated with sodium hydroxide to denature the cDNA in the plaques. The conditions for denaturation were the same as those described in 7.(1).
(3) The denatured cDNA was heat-treated for 2 hours at 75 °C to have it fixed, and was used for hybridization. The probe used in the hybridization was a part of the HRTI gene whose base sequence had been determined in 8., that part being randomly labeled with ³²P-dCTP by means of a random labeling kit of Boehringer Mannheim. The conditions for hybridization and the conditions for washing followed those described in the literature.
(4) As a result of hybridization, a gene having a longer sequence than the sequence of the probe was obtained from plaques corresponding to positive signals obtained from the cDNA fixed to the filter.

Thus, a full-length gene was obtained which corresponded to the HRPI gene described above.

### [Example 2] Determination of Amino Acid Sequence of Hepatic Cancer Specific Protein HRPI and Gene Encoding the Protein

### 1. Large-scale preparation of gene

The following procedure was performed on HRPI gene to prepare the gene on a large scale.
(1) The phage recovered from the plaques formed on the NZY agar medium in 11.(4) or (5) of Example 1 was suspended in SM solution.
(2) The suspension (50 µl) of (1) and 20 µl of Y1090r-*E. coli* were mixed, and allowed to stand for 15 minutes at 37 °C.
(3) Then, the mixed solution in (2) was transferred to a 10 ml NZY medium containing 100 µg/ml ampicillin, and cultured for 6 hours at 37 °C.
(4) The culture was centrifuged for 5 minutes at 8,000 rpm, and the supernatant was recovered.
(5) To the supernatant, 1 ml of 5 M NaCl and 1.1 g of polyethylene glycol 6000 were added and dissolved.
(6) The solution was placed on ice for 1 hour, and then centrifuged for 20 minutes at 4 °C at 10,000 rpm.
(7) The precipitate was recovered and suspended in 700 µl of SM solution.
(8) Chloroform (500 µl) was added to the suspension, and the mixture was stirred to dissolve the remaining *E. coli* cells.
(9) The solution was centrifuged for 10 minutes at 5,000 rpm, and the aqueous phase was recovered.
(10) To the aqueous phase, 1 mg/ml RNaseA and 5 mg/ml DNaseI (both from Sigma) were added in an amount of 1 µl each. After the mixture was allowed to stand at 37 °C for 1 hour, 600 µl of 20% polyethylene glycol 6000 (0.8 M NaCl) was added. The mixture was allowed to stand for 30 minutes on ice.
(11) The system was centrifuged for 20 minutes at 4 °C at 15,000 rpm, and then the precipitate was recovered.
(12) To the precipitate, 500 µl of SM solution, 50 µl of 5 M NaCl and 50 µl of 0.5 M EDTA were added, and 400 µl of phenol was further added. The mixture was stirred to dissolve the phage and liberate cDNA.
(13) The solution was centrifuged for 5 minutes at 15,000 rpm at room temperature, and then the aqueous phase was recovered. To the liquid, 1 ml of ethanol was added, and the mixture was centrifuged for 20 minutes at 15,000 rpm. The liquid phase was discarded.
(14) The precipitate was washed with 1 ml of 70% ethanol, and dissolved in 100 µl of TE solution (10 mM Tris-Cl pH 8.0, 1 mM EDTA) to obtain a DNA solution.

### 2. Insertion of HRPI gene into vector

HRPI gene was inserted into a vector by the procedure described below.
(1) A DNA cleavage system of the composition described below was prepared to cut DNA with the restriction enzyme NotI (Takara Shuzo).

| | |
|---|---|
| DNA solution (prepared in 1.) | 20 µl |
| 0.1% BSA | 10 µl |
| 0.1% TritonX100 | 10 µl |
| NotI (Takara Shuzo) | 2 µl |
| RNase A (Nippon Gene) | 1 µl |
| 10xH buffer (Takara) | 10 µl |
| Sterilized water | 47 µl |
| | Total 100 µl |

Reaction temperature: 37 °C
Reaction time: 4 hours

(2) Then, the system was electrophoresed on a 0.7% NuSieve (registered trademark) GTG Agarose (Takara Shuzo), and DNA appearing around 1.6 kbp was cut out. This DNA was recovered by means of GENE CLEAN II (registered trademark, Funakoshi) in accordance with its instruction manual.
(3) pBluescriptII (Stratagene) into which DNA would be incorporated was cleaved with NotI, and then dephosphorylated.
(i) Cleavage with NotI was carried out using the following system:

| | |
|---|---|
| pBluescriptII (1 µg/ l) | 3 µl |
| 10xH buffer | 2 µl |
| 0.1% BSA | 2 µl |
| 0.1% Tritonx100 | 2 µl |
| NotI | 2 µl |
| Sterilized water | 10 µl |
| | Total 20 µl |

Reaction temperature: 37 °C
Reaction time: overnight

(ii) Then, 2 µl of 1 M Tris (pH 8.0) was added, and 1 µl of Bacterial Alkaline Phosphatase (Takara Shuzo) was added, and the system was allowed to stand for 1 hour at 65 °C.
(iii) Then, phenol/CHCl₃ extraction was performed twice in accordance with a standard method to deactivate the enzymes. After purification by ethanol precipitation, the precipitate was dissolved in a TE solution to a concentration of 100 ng/µl.
(4) The DNA obtained in (2) and the pBluescriptII obtained in (3) were reacted using the following system to insert the DNA into the vector:

| | |
|---|---|
| DNA (prepared in (2)) | 5 µl |
| NotI-cleaved pBluescriptII (prepared in (3)) | 1 µl |
| 10-fold ligation buffer (Nippon Gene) | 2 µl |
| T4 ligase (Nippon Gene) | 1 µl |
| Sterilized water | 11 µl |
| | Total 20 µl |

Reaction temperature: 16 °C
Reaction time: 2 hours

### 3. Incorporation of gene into E. coli

In accordance with a standard method, the reaction mixture containing the vector into which HRPI gene had been inserted (prepared in 2.) was completely mixed with competent cells of *E. coli* JM109 (Takara Shuzo). The mixture was reacted for 30 minutes on ice, for 45 seconds at 42 °C, and for 3 minutes on ice. Then, 900 µl of SOC culture medium was added, and the mixture was allowed to stand for 1 hour at 37 °C to incorporate the vector into the *E. coli* JM109. Then, the *E. coli* JM109 was recovered.

The recombinant *E. coli* having this HRPI gene introduced therein was deposited at the National Institute of Bioscience and Human Technology (locating at 1-3, Higashi 1-chome, Tsukuba-shi, Ibaraki-ken, Japan), Agency of Industrial Science and Technology, Ministry of International Trade and Industry, Japan, with the accession number FERM P-15164. Then, the microorganism was transferred to international deposition on September 11, 1996 (Accession No. FERM BP-5663).

### 4. Base sequencing of gene

(1) The *E. coli* JM109 recovered in 3. was sprinkled on a LB agar medium (containing 100 µg/ml ampicillin, 0.1 mM IPTG, 0.004% X-gal), and incubated for 16 hours at 37 °C.
(2) Of the colonies formed, white colonies were inoculated into 2 ml of LB medium (containing 100 µg/ml ampicillin), and cultured for 16 hours at 37 °C.
(3) Then, the culture was centrifuged for 1 minute at 12,000 rpm to harvest the cells, and a plasmid DNA solution was recovered by means of Magic Miniprep (registered trademark, Promega).
(4) The recovered DNA was sequenced using a Dyeterminator FS Sequencing Kit (Perkin-Elmer) in the manner described below.
(i) According to the instruction manual to the Kit, the DNA was made into a sequencing sample.
(ii) According to the instruction manual to the Kit, PCR of the DNA was carried out in the following system:

| | |
|---|---|
| Template DNA | 600 µl |
| Primer DNA | 3.2 pmol |
| Sequencing mix | 8 µl |

Sterilized distilled water was added to bring the reaction solution to a total of 20 µl. The primers prepared by custom synthesis were used.
(iii) The PCR product was extracted with phenol/chloroform.
(iv) The extract was precipitated with ethanol, purified, and dried.
(v) The product was dissolved into a 3 µl solution of 3% blue dextran, 50 mM EDTA, and 80% formamide, and denatured for 2 minutes at 95 °C. Then, upon cooling the solution was made into a sequencing sample.
(vi) The obtained sample, 373 Stretch DNA, was analyzed using a 373 Stretch DNA Sequencer (Perkin-Elmer), and the base sequence was determined.

The base sequence of the HRPI gene is shown in SEQ ID NO: 2. The procedure was repeated: the DNA recovered in 4. (3) was sequenced. This confirmed that the base sequence of the HRPI gene was the sequence as described in SEQ ID NO: 2 in the Sequence Listing.

### 5. Determination of amino acid sequence

The amino acid sequence of HRPI was determined by the base sequences determined in 4. The amino acid sequence of HRPI is shown as SEQ ID NO: 1 in the Sequence listing.

### [Example 3] Confirmation of Possibility for Diagnosis of Hepatic Cancer at Gene Expression Level

The analysis of HRPI gene by Northern blot hybridization was made in accordance with the method using formaldehyde denatured gel electrophoresis described in Current Protocols in Molecular Biology (1987, Greene Publishing Associates and Wiley-Interscience), Chapter 4. PolyA RNAs used in the analysis were two kinds: (i) polyA RNA derived from a normal liver and that from hepatic cancer 7 months after DEN administration, and (ii) all polyA RNAs of hepatic cancer samples prepared in Example 1. The amount of the polyA RNA used was 500 ng for each sample.
(1) The HRPI gene carried by pBluescriptII in I.2. of Example 2 was cut out of the vector by treatment with a restriction enzyme.
(2) Then, the restriction enzyme-treated solution was subjected to agarose gel electrophoresis to separate the desired HRPI gene.
(3) The HRPI gene separated in (2) was purified with GENE CLEAN II (registered trademark, Funakoshi).
(4) The purified HRPI gene was labeled by a Random Primed DNA Labeling Kit (Boehringer Mannheim) using α-P³²dCTP (Amersham) in accordance with its instruction manual to make a ³²P-dCTP-labeled HRPI gene probe.
(5) Systems using the normal liver and the 7-month hepatic cancer were each analyzed by Northern blot hybridization using the probe prepared in (4). As a result, HRPI gene was detected as a gene significantly increased in hepatic cancer. This finding showed that hepatic cancer and normal liver could be distinguished, namely, the occurrence of hepatic cancer could be monitored, and further hepatic cancer could be diagnosed, by using the HRPI gene.
(6) Then, systems using all polyA RNAs of the hepatic cancer samples prepared in 1. were each analyzed by Northern blot hybridization using the same probe. As the induction of cancer progresses, a significant increase in HRPI gene was noted (Fig. 2). This finding showed that the use of HRPI gene would permit the distinction of hepatic cancer at its initial stage, i.e., the monitoring of progress of hepatic cancer, and further the early diagnosis of hepatic cancer.

### [Example 4] Expression of HRPI Partial Protein

### 1. Construction of recombinant E. coli

A partial length clone (the portion encoding from the 185th to 299th amino acids of the sequence as described in SEQ ID NO: 1 in the Sequence Listing) of the HRPI gene obtained in Example 1.8. was integrated into pET3a vector having a histidine tag incorporated therein (Fig. 3). Then, the vector was introduced into *E. coli* BL21 (DE3) pLysS.

### 2. Preparation of HRPI

(1) The *E. coli* produced in 1. was cultured in a LB medium containing 100 µg/ml ampicillin. When turbidity measured with a spectrophotometer (Beckman) reached 0.5 at a wavelength of 600 nm, IPTG was added to provide a concentration of 0.5 mM, inducing the expression of HRPI protein.
(2) Two hours later, *E. coli* cells were recovered, and suspended in Lysis Buffer. The suspension was sonicated, and centrifuged for 15 minutes at 18,000 rpm at 4 °C with 50.2 Ti rotor using Beckman Optima XL-80.
(3) Then, the precipitate was dissolved in 6 M guanidine hydrochloride, and purified with Ni-agarose (Qiagen) in accordance with its instruction manual.

### 3. SDS-PAGE electrophoresis

The purified sample was subjected to SDS-polyacrylamide electrophoresis, and stained with Coomassie brilliant blue to make sure that purification was successful. The results are shown in Fig. 4.

### [Example 5] Expression of HRPI Full-Length Protein 1. Large-scale preparation of HRPI gene

### (1) Construction of recombinant vector

HRPI gene was integrated into a pBlueBacIII vector (Invitrogen) having a histidine tag incorporated therein as shown in Fig. 5. The details are provided below.
1) The pBluescriptII vector having HRPI gene inserted therein that was constructed in Example 2.2. was amplified by the PCR method described in Current Protocols in Molecular Biology (Greene Publishing Associates and Wiley-Interscience, 1987), Chapter 15. In the amplification, primers of the base sequences of the following formulae (1) and (2) were used, and the base sequence encoding the first methionine portion of HRPI gene was used as NdeI recognition site.
   5' GAA TTC CAT ATG TTC TAT ATA CAG AGT TCT GAG GC 3' Formula (1)
   5' TCC AGT TAG TGA CGT CTG ATG 3' Formula (2)
2) The amplified gene was cleaved with Ndel and BamHI (both from Takara Shuzo) under the conditions exactly described in their brochures.
3) Separately, HRPI gene on the pBluescriptII prepared in Example 2. 2. was cleaved with BamHI and SacI (both from Takara Shuzo) under the conditions exactly described in their brochures. With respect to each of the cleaved genes, only the desired portion was purified using agarose gel electrophoresis and GENE CLEAN II (registered trademark, Funakoshi).
4) The genes prepared in 2) and 3) were ligated together by means of Ligation Pack (Nippon Gene) in accordance with its instruction manual.
5) EcoRI/NdeI/EcoRI adapter (5' AATTCCATATGG 3') was synthesized by a DNA synthesizer, and phosphorylated with T4 polynucleotide kinase (Takara Shuzo) in accordance with its instruction manual.
6) BluescriptII was cleaved with EcoRI, and the EcoRI/Ndel/EcoRI adapter produced in 5) was integrated into the cleavage site.
7) The Ndel and SacI of the vector constructed in 6) were cleaved, and the gene prepared in 4) was integrated into the cleavage site.
8) The above vector was introduced into *E. coli* JM109 in accordance with a standard method, and the *E. coli* having the vector inserted therein was cultured in a LB medium (containing 100 µg/ml ampicillin).
9) From the cultured *E. coli* cells, the desired vector was purified by use of Magic Miniprep (registered trademark, Promega) in accordance with its instruction manual.
10) The vector was cut with NdeI, and then joined to a NdeINcoI linker (5' TATCCATGG 3') by means of Ligation Pack to turn both cut ends of the vector into NcoI.
11) The NcoI sites of the HRPI gene portion of the vector were cleaved with a restriction enzyme to prepare HRPI gene with NcoI ends.
12) After agarose gel electrophoresis of the gene by a standard method, only the desired portion was purified with GENE CLEAN II.
13) pBlueBacIII vector (Invitrogen) having a histidine tag incorporated therein as shown in Fig. 5 was cut with NcoI by a standard method, and then dephosphorylated.
14) The HRPI gene purified in 10) and the vector produced in 11) were ligated together by means of Ligation Pack (Nippon Gene) in accordance with its instruction manual.

Confirmation of the gene integrated into the vector or *E. coli* at each step described above was performed by reading the base sequence of the insert.

### (2) Large-scale preparation of HRPI gene

The HRPI gene-integrated plasmid was prepared on a large scale by the method described in Molecular Cloning Second Edition, Cold Spring Harbor Laboratory Press, 1989), Chapter 1. The plasmid was purified by ultracentrifugation described there. This procedure was performed 3 times.

### 2. Expression of HRPI full-length protein

(1) The mass produced plasmid was cotransfected into Sf9 cells by lipofection (DO TAP (registered trademark, Boehringer Mannheim)).
(2) The resulting recombinant virus was again introduced into Sf9 cells to cause large-scale expression. The handling of the Sf9 cells after lipofection, production of the recombinant virus, and large-scale expression of the protein were all performed in accordance with the instruction manual of MAXBAC (registered trademark, Invitrogen).
(3) The Sf9 cells having expressed HRPI were suspended in SDS sample buffer, boiled for 5 minutes, and then subjected to SDS-PAGE electrophoresis. Then, the electrophoresed sample was stained with Coomassie brilliant blue, whereby a band for HPRI was detected. Fig. 6 shows the results of the SDS-PAGE electrophoresis.

### [Example 6] Preparation of anti-HRPI Antibody

### 1. Preparation of anti-HRPI antibody

The HRPI partial protein prepared in Example 4 was inoculated into rabbits for immunization in accordance with the method described in Antibodies, A Laboratory Manual (Cold Spring Harbor Laboratory Press, 1988), Chapter 5, to produce an anti-HRPI antibody.

### 2. Western blot

The method described in Current protocols in molecular biology (Greene Publishing Associates and Wiley-Interscience, 1987), Chapter 1 was followed.
(1) Western blotting of the HRPI partial protein prepared in Example 4 and the HRPI full-length protein prepared in Example 5 was performed.
(2) Then, the anti-HRPI antibody was allowed to react with each of the HRPI partial protein and the HRPI full-length protein. The complexes were each allowed to react with alkaline phosphatase-labeled anti-rabbit IgG antibodies as secondary antibodies. The alkaline phosphatase was further allowed to react with a substrate (NBT, BCIP (both products of Promega)) for color development (Fig. 7A). The anti-HRPI antibody was confirmed to react with HRPI. Fig. 7A shows that recombinant HRPI has a larger molecular weight by the size of the histidine tag.

### [Example 7] Investigation of Use of HRPI in Diagnosing Hepatic Cancer

(1) One gram each of the various hepatic cancer tissues prepared in Example 1 was homogenized in 5 ml of 2xSDS sample buffer, and boiled for 3 minutes to obtain the hepatic cancer tissue extract.
(2) The extract was subjected to 12.5% SDS-PAGE electrophoresis, and then Western blotted. The amounts of protein were unified by staining the gels, separately electrophoresed similarly, with Coomassie brilliant blue and by comparing the colors.
(3) Then, the extract was allowed to react with the anti-HRPI antibody prepared in Example 6. The complex was allowed to react with alkaline phosphatase-labeled anti-rabbit IgG antibodies as secondary antibodies. The alkaline phosphatase was further allowed to react with substrates (NBT, BCIP (both from of Promega)) for color development (Fig. 7B). As with the results of Northern blot hybridization, significant increases in HRPI protein were confirmed in hepatic cancer and in the process of hepatic cancer occurrence. That is, the antibody was able to distinguish between hepatic cancer and normal liver, and also to distinguish between a liver in the process of carcinogenesis and a normal liver. Thus, it was confirmed to be usable in the monitoring of hepatic cancer occurrence and the early diagnosis of hepatic cancer.

### [Example 8] Confirmation of HRPI Gene Distribution in Tissues

To confirm the distribution of the gene in various tissues, Rat MTN Blot (registered trademark, Clontech) was used. This product is a commercially available membrane blotted with polyA RNAs of rat tissues to be used in the above-mentioned Northern blot hybridization. This membrane was subjected to Northern blot hybridization by the method described in Molecular Cloning Second Edition, (Cold Spring Harbor Laboratory Press, 1989) pp. 7.3-7.84 in accordance with the product's instruction manual with the use of the aforementioned HRPI gene probe.

The results with the HRPI gene probe are shown in Fig. 8. These results indicate that the HRPI gene was intensely expressed in the liver and kidney, and in the lung as well.

### Industrial Applicability

The detection of HRPI or the HRPI gene, which have been revealed by the present invention, in the liver permits the monitoring of the progress of hepatic cancer, and diagnosis of hepatic cancer, especially the early diagnosis of hepatic cancer.

Furthermore, the use of a part or the whole of the HRPI gene, which has been revealed by the present invention makes it possible to develop diagnostic agents for, or methods of diagnosing the onset of hepatic cancer at the gene expression level.

Moreover, the use of the anti-HRPI antibody according to the present invention permits the immunohistological diagnosis of hepatic cancer occurrence.

In the future, the gene, antisense genes to the gene, or antibodies against the gene can be expected to find use in therapeutic drugs for hepatic cancer as well as in the methods of treatment of hepatic cancer.

## Claims

1. A protein represented by an amino acid sequence described as SEQ ID NO: 1 in the Sequence Listing.

2. A protein comprising an amino acid sequence described as SEQ ID NO: 1 in the Sequence Listing wherein one or more amino acids have been added to, deleted from, or replaced for, and whose expression in a hepatic tissue is increased in cancer cells compared with normal cells.

3. DNA comprising a base sequence encoding the protein of either claim 1 or claim 2.

4. DNA comprising a base sequence encoding at least part of the protein of either claim 1 or claim 2, and hybridizing to RNA encoding the whole length of the protein.

5. DNA comprising a base sequence described as SEQ ID NO: 2 in the Sequence Listing.

6. DNA comprising a base sequence ranging from the 25th base A to the 912th base A in the base sequence described as SEQ ID NO: 2 in the Sequence Listing.

7. DNA comprising consecutive 12 bases or more in a base sequence encoding the protein of either claim 1 or claim 2, and having a GC content of from 30 to 70%.

8. The DNA of any one of claims 3 to 7, which has been chemically modified.

9. Antisense DNA to the DNA of any one of claims 3 to 7.

10. RNA comprising a base sequence encoding the protein of either claim 1 or claim 2.

11. RNA comprising consecutive 12 bases or more in a base sequence encoding the protein of either claim 1 or claim 2, and having a GC content of from 30 to 70%.

12. The RNA of either claim 10 or claim 11, which has been chemically modified.

13. Antisense RNA to the RNA of either claim 10 or claim 11.

14. An antibody specifically reactive to the protein of either claim 1 or claim 2.

15. A method for detecting the protein of either claim 1 or claim 2, the method using the antibody of claim 14.

16. A method for detecting RNA encoding the protein of either claim 1 or claim 2, the method using the DNA of any one of claims 3 to 8 as a probe.
